# EUROPÄISCHE PATENTANMELDUNG

(11) **EP 4 279 607 A1**
(43) Veröffentlichungstag der Anmeldung: **22.11.2023**
(21) Anmeldenummer: 22174472.5
(22) Anmeldetag: 20.05.2022
(51) Int. Cl.: C12Q 1/37

(54) **ENZYM-VERSTÄRKTER ADAMTS-13 AKTIVITÄTSTEST**

(71) Anmelder: Siemens Healthcare Diagnostics Products GmbH, 35041 Marburg (DE)
(72) Erfinder: Schwarz, Herbert, 35102 Lohra (DE); Vitzthum, Frank, 35216 Biedenkopf (DE)

(57) **Zusammenfassung**

Die vorliegende Erfindung bezieht sich auf ein Verfahren zur Bestimmung der ADAMTS-13 Aktivität in einer Probe, umfassend das Inkontaktbringen der Probe mit einem von Willebrand Faktor (VWF)-Polypeptid, das an ein Reporterenzym, wie z.B. Glukose-6-Phosphat-Dehydrogenase (G6PDH), gekoppelt ist.

## Beschreibung

Die vorliegende Erfindung bezieht sich auf ein Verfahren zur Bestimmung der ADAMTS-13 Aktivität in einer Probe, umfassend das Inkontaktbringen der Probe mit einem inhibierten Reporterenzymkomplex, der ein von Willebrand Faktor (VWF)-Polypeptid mit einer ADAMTS-13 Spaltstelle, das an ein Reporterenzym gekoppelt ist, umfasst. Darüber hinaus betrifft die Erfindung einen Kit umfassend Reagenzien, die einen solchen inhibierten Reporterenzymkomplex, ein Reporterenzymsubstrat und optional einen oder mehrere Cofaktoren für das Reporterenzym enthalten. Ferner betrifft die Erfindung die Verwendung eines solchen inhibierten Reporterenzymkomplexes für die Bestimmung der ADAMTS-13 Aktivität in einer biologischen Probe.

ADAMTS-13 (A Disintegrin And Metalloproteinase with a Thrombospondin type 1 motif, member 13) ist ein Enzym, das den von Willebrand Faktor (VWF) in vivo abbaut. Es gibt kongenitale und erworbene Defizienzen der ADAMTS-13-Enzymaktivität, die zu Erkrankungen, wie z.B. der thrombotisch-thrombozytopenischen Purpura (TTP) führen. Ein bekannter Test zur Messung der ADAMTS-13 Aktivität in Plasmaproben funktioniert derart, dass die Menge der abgebauten VWF-Aktivität mit einem Ristocetin-Kofaktor-Test bestimmt wird (WO 2004/005451 A2). Dazu wird zu einer relativ geringen Menge an Probe VWF als Substrat zugegeben, ADAMTS-13 aktiviert und dann während einer sehr langen Inkubationszeit (über Nacht) VWF abgebaut. Entscheidend ist bei diesem Ansatz, dass durch die Probe selbst möglichst wenig VWF mit in den Ansatz kommt, da dessen Aktivität im Plasma hohen Schwankungen unterliegen kann. Weiterhin darf mit dem Substrat keine ADAMTS-13-Aktivität mit in den Ansatz gelangen, weil nur die ADAMTS-13-Aktivität aus der Probe gemessen werden soll.

In analoger Weise kann ein Kollagenbindungstest verwendet werden, um den Verlust der großen VWF-Multimere zu detektieren (WO 0050904 A1). Auch dieser ADAMTS-13-Aktivitätstest hat sehr lange Inkubationszeiten, weil nur wenig Plasmaprobe eingesetzt werden kann und weil nativer VWF sehr langsam abgebaut wird.

Eine Verbesserung des ADAMTS-13-Aktivitätstests wurde durch die Verwendung von hitzebehandeltem VWF-Mangelplasma erreicht. Dadurch konnte die Inkubationszeit auf 60 Minuten reduziert werden (siehe Kostousov et al., 2006, Thromb Res; 118(6):723-31). Ein weiterer Test aus dem Stand der Technik benutzt VWF-Polypeptide als Substrat für ADAMTS-13. Die Spaltprodukte werden quantitativ erfasst und mit der ADAMTS-13-Aktivität korreliert (siehe US2007/065895 A1).

Ein weiterer Test wurde in US 2005/186646 A1 beschrieben. Er verwendet Polypeptide, die die Spaltungsstelle für ADAMTS-13 enthalten, als Substrat. Gemessen wird die Entstehung der Fragmente.

Ein weiteres Verfahren wurde von Kokame et al., 2005, Br. J. Haematol. 129:93-100 beschrieben. Dieser Ansatz nutzt ein Polypeptid-Substrat für die Bestimmung der ADAMTS-13 Aktivität, wobei das Substrat aus 73 Aminosäureresten aus der A2 Domäne von VWF, insbesondere aus D1596 bis R1668 der A2 Domäne, besteht und als VWF73 bezeichnet wird. Für den Nachweis wurde es mit FRET (Fluoreszenz-Resonanzenergietransfer)-Reagenzien modifiziert. Ein ähnlicher Ansatz ist in WO 2013/071168 A1 beschrieben, bei dem durch Aminosäureaustausche oder -deletionen in dem VWF-Polypeptid eine effektivere Spaltung des VWF-Polypeptids durch ADAMTS-13 erzielt wird. Der Nachweis der Spaltung erfolgt ebenfalls durch die FRET Methode. Allerdings bieten klassische Gerinnungsanalyzer normalerweise nicht die Möglichkeit der FRET Detektionstechnologie, sodass diese Ansätze in derartigen Geräten nicht anwendbar sind.

Kommerziell verfügbare Teste (z.B. Technozym ADAMTS-13 Activity Test oder HemosIL Acustar ADAMTS-13 Aktivitätassay) verwenden das VWF73-Polypeptid im Rahmen eines Antikörpernachweisverfahrens. Das VWF73-Polypeptid ist dabei auf einer Festphase immobilisiert, die mit der Probe in Kontakt gebracht wird. Die durch die ADAMTS-13 Aktivität entstehenden VWF-Fragmente werden dann von Antikörpern erkannt. Der Nachteil dieser heterogenen Teste ist die Notwendigkeit von Waschschritten. Sie sind deshalb für die Abarbeitung in Routine-Gerinnungsanalyzern ungeeignet.

Der vorliegenden Erfindung liegt somit die Aufgabe zugrunde, Verfahren und Mittel bereitzustellen, welche es erlauben, die ADAMTS-13 Aktivität in einer Probe effizient, zeitsparend und in automatisierbarer Weise nachzuweisen.

Diese Aufgabe wird durch die Gegenstände der unabhängigen Ansprüche gelöst. In den abhängigen Ansprüchen sind weitere vorteilhafte Aspekte der Erfindung reflektiert.

Im Wesentlichen wird die erfindungsgemäße Aufgabe dadurch gelöst, dass eine Probe mit einem inhibierten Reporterenzymkomplex, der ein von Willebrand Faktor (VWF)-Polypeptid mit einer ADAMTS-13 Spaltstelle und ein Reporterenzym umfasst und der durch die enzymatische Spaltung des VWF-Polypeptids durch ADAMTS-13 aktivierbar ist, und mit einem Reporterenzymsubstrat zu einem Reaktionsgemisch vermischt wird und die Menge des von dem durch die ADAMTS-13 Aktivität der Probe aktivierten Reporterenzym umgesetzten Reporterenzymsubstrats in dem Reaktionsgemisch gemessen wird. Die so gemessene Menge des umgesetzten Reporterenzymsubstrats ist proportional zur ADAMTS-13 Aktivität in der Probe.

Dies hat den Effekt, dass die ADAMTS-13 Aktivität in einer Probe effizient und zeitsparend bestimmt werden kann und dass das Verfahren in einem homogenen Testformat, also ohne Waschschritte durchgeführt werden kann und damit einer automatisierten Abarbeitung auf gängigen Gerinnungsanalyzern zugänglich ist.

Gegenstand der vorliegenden Erfindung ist also ein Verfahren zur Bestimmung der ADAMTS-13 Aktivität in einer Probe, das Verfahren umfassend die folgenden Schritte:
a. Bereitstellen eines Reaktionsgemischs durch Vermischen der Probe mit
   i. einem inhibierten Reporterenzymkomplex, der ein von Willebrand Faktor (VWF)-Polypeptid mit einer ADAMTS-13 Spaltstelle und ein Reporterenzym umfasst und der durch die enzymatische Spaltung des VWF-Polypeptids durch ADAMTS-13 aktivierbar ist und
   ii. einem Reporterenzymsubstrat;
b. Messen der Menge des von dem aktivierten Reporterenzym umgesetzten Reporterenzymsubstrats in dem Reaktionsgemisch,
wobei die gemessene Menge des umgesetzten Reporterenzymsubstrats proportional zur ADAMTS-13 Aktivität in der Probe ist.

Das Reporterenzym kann Lysozym, Malat-Dehydrogenase, beta-Galaktosidase, alpha-Amylase oder Glukose-6-Phosphat-Dehydrogenase (G6PDH) oder eine funktionelle Variante davon sein. Ein besonders bevorzugtes Reporterenzym ist Glukose-6-Phosphat-Dehydrogenase (G6PDH). Optional können einer oder mehrere Cofaktoren verwendet werden oder anwesend sein.

Die Messung des umgesetzten Reporterenzymsubstrats kann mittels einer spektrophotometrischen, luminometrischen (z.B. Fluoreszenzbestimmung) oder elektrochemischen Methode erfolgen.

In einer bevorzugten Ausführungsform sind das VWF-Polypeptid mit einer ADAMTS-13 Spaltstelle und das Reporterenzym in dem inhibierten Reporterenzymkomplex kovalent miteinander verknüpft. Bevorzugt ist eine Verknüpfung über einen Spacer, am meisten bevorzugt über einen Spacer mit 3 bis 10 Atomen.

Weiter bevorzugt ist, dass das VWF-Polypeptid die A2 Domäne des VWF umfasst.

In besonders bevorzugten Ausführungsformen der Erfindung besitzt das VWF-Polypeptid die Aminosäuresequenz einer der SEQ ID NO: 1 bis SEQ ID NO: 24.

Das VWF-Polypeptid kann C-terminal mit einem Epitop-Tag versehen sein. Bevorzugte Epitop-Tags sind Biotin, FITC, Streptavidin, GST, His, Flag, ACP, ELK16, HA oder MBP.

In dem erfindungsgemäßen Verfahren bewirkt die ADAMTS-13 Aktivität im Reaktionsgemisch die Aufhebung der durch die Kopplung des VWF-Polypeptids an das Reporterenzym verursachten Inhibierung des Reporterenzyms, indem das ADAMTS-13 Enzym das gekoppelte VWF-Polypeptid proteolytisch spaltet. Hierbei ist besonders bevorzugt, dass die durch das gekoppelte VWF-Polypeptid verursachte Inhibierung des Reporterenzyms durch die Bindung eines Antikörpers oder eines anderen spezifischen Bindungspartners, beispielsweise eines Aptamers oder Affimers, an das VWF-Polypeptid verstärkt wird. Vorzugsweise umfasst der inhibierte Reporterenzymkomplex also zusätzlich einen an das VWF-Polypeptid spezifisch gebundenen Bindungspartner aus der Gruppe Antikörper, Aptamer und Affimer.

In einer spezifischen Ausführungsform ist dabei der spezifisch gebundene Bindungspartner an ein Epitop-Tag am VWF-Polypeptid gebunden.

Bei der Probe kann es sich um eine Körperflüssigkeit, wie z.B. Vollblut, Blutplasma, Blutserum, Speichel, Liquor oder Tränenflüssigkeit, handeln.

Ein weiterer Gegenstand der vorliegenden Erfindung ist ein Kit, umfassend ein erstes Reagenz enthaltend einen inhibierten Reporterenzymkomplex, der ein VWF-Polypeptid mit einer ADAMTS-13 Spaltstelle und ein Reporterenzym umfasst und der durch die enzymatische Spaltung des VWF-Polypeptids durch ADAMTS-13 aktivierbar ist, und ein zweites Reagenz enthaltend ein Reporterenzymsubstrat. Optional umfasst das Kit einen oder mehrere Cofaktoren für das Reporterenzym und/oder für die ADAMTS-13 Protease. Der oder die Cofaktoren können in dem zweiten Reagenz enthalten sein oder in einem separaten dritten Reagenz.

Noch ein weiterer Gegenstand der vorliegenden Erfindung ist die Verwendung eines inhibierten Reporterenzymkomplexes, der ein VWF-Polypeptid mit einer ADAMTS-13 Spaltstelle und ein Reporterenzym umfasst und der durch die enzymatische Spaltung des VWF-Polypeptids durch ADAMTS-13 aktivierbar ist, für die Bestimmung der ADAMTS-13 Aktivität in einer Probe.

### KURZE BESCHREIBUNG DER FIGUREN

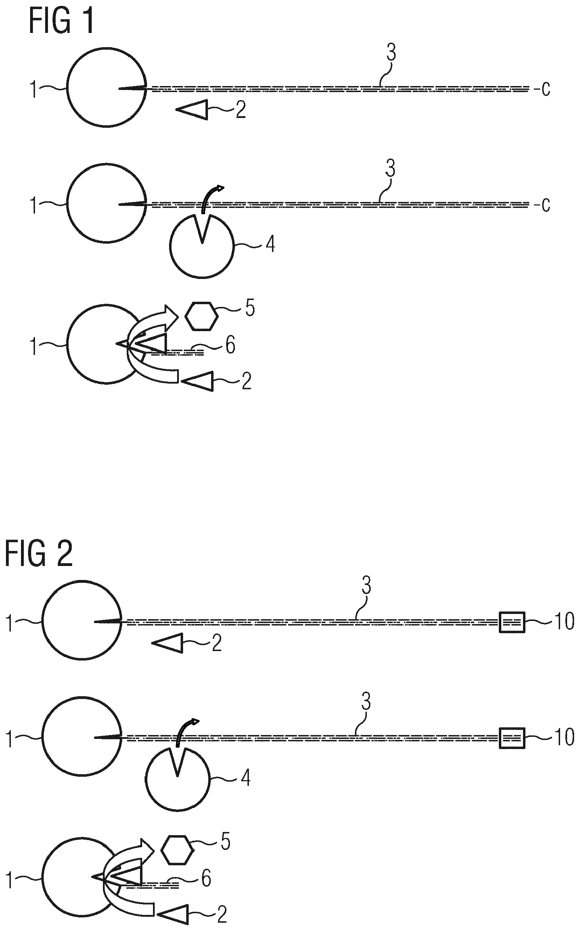

- **FIG. 1**: zeigt eine Ausführungsform des erfindungsgemäßen Verfahrens, in der ein Reporterenzym (1), an das ein VWF-Polypeptid (3) N-terminal gekoppelt ist. Die Kopplung des VWF-Polypeptids (3) inhibiert die Aktivität des Reporterenzyms (1) und verhindert damit die Umsetzung eines Reporterenzymsubstrats (2). Mittels proteolytischer Spaltung des VWF-Polypeptids (3) durch ADAMTS-13 (4) wird das VWF-Polypeptid (3) gespalten, und es bleibt nur Fragment (6) am Reporterenzym übrig. Dies bewirkt, dass die Inhibierung des Reporterenzyms (1) aufgehoben oder stark reduziert wird, so dass das Reporterenzymsubstrat (2) nun mit dem Reporterenzym (1) interagieren kann und in ein nachweisbares Produkt (5) umgewandelt wird.
- **FIG. 2**: zeigt eine weitere Ausführungsform des erfindungsgemäßen Verfahrens, die im Wesentlichen dem in Fig. 1 gezeigten Verfahren entspricht mit dem Unterschied, dass das VWF-Polypeptid (3) C-terminal mit einem Epitop-Tag (10) verknüpft ist. Dieses Epitop-Tag kann zur Regulierung der Inhibierung des Reporterenzyms (1) oder für Zwecke der Abtrennung nach einem proteolytischen Schritt verwendet werden.

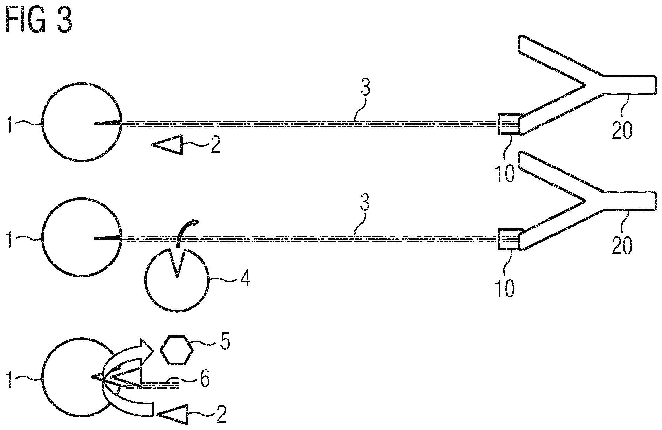

- **FIG. 3**: zeigt eine weitere Ausführungsform des erfindungsgemäßen Verfahrens, die im Wesentlichen dem in Fig. 2 gezeigten Verfahren entspricht mit dem Unterschied, dass an das C-terminale Epitop-Tag (10) des VWF-Polypeptids (3) ein Antikörper oder ein anderer spezifischer Bindungspartner (20) gebunden ist. Diese Interaktion kann zur Regulierung der Inhibierung des Reporterenzyms (1) oder für Zwecke der Abtrennung nach einem proteolytischen Schritt verwendet werden.

Obwohl die vorliegende Erfindung in Bezug auf bestimmte Ausführungsformen beschrieben wird, ist diese Beschreibung nicht in einem einschränkenden Sinne aufzufassen.

Bevor beispielhafte Ausführungsformen der vorliegenden Erfindung im Detail beschrieben werden, werden Definitionen gegeben, die für das Verständnis der vorliegenden Erfindung wichtig sind.

Wie in dieser Beschreibung und in den beigefügten Ansprüchen verwendet, schließen die Singularformen von "ein" und "eine" auch die jeweiligen Pluralformen ein, sofern der Kontext nicht eindeutig etwas anderes vorschreibt.

Im Zusammenhang mit der vorliegenden Erfindung bezeichnen die Begriffe "ungefähr" und "etwa" ein Genauigkeitsintervall, das ein Fachmann verstehen wird, um die technische Wirkung des betreffenden Merkmals noch sicherzustellen. Der Begriff gibt typischerweise eine Abweichung von dem angegebenen Zahlenwert von ±20 %, vorzugsweise ±15 %, stärker bevorzugt ±10 % und noch stärker bevorzugt ±5 % an.

Es versteht sich, dass der Begriff "umfassend" nicht einschränkend ist. Für die Zwecke der vorliegenden Erfindung wird der Begriff "bestehend aus" oder "im Wesentlichen bestehend aus" als eine bevorzugte Ausführungsform des Begriffs "umfassend aus" angesehen.

Wenn im Folgenden eine Gruppe so definiert wird, dass sie mindestens eine bestimmte Anzahl von Ausführungsformen umfasst, soll dies auch eine Gruppe umfassen, die vorzugsweise nur aus diesen Ausführungsformen besteht.

Darüber hinaus werden die Begriffe "(i)", "(ii)", "(iii)" oder "(a)", "(b)", "(c)", "(d)" oder "erste", "zweite", "dritte" usw. und dergleichen in der Beschreibung oder in den Ansprüchen zur Unterscheidung zwischen ähnlichen Elementen und nicht notwendigerweise zur Beschreibung einer sequentiellen oder chronologischen Reihenfolge verwendet.

Es versteht sich, dass die so verwendeten Begriffe unter geeigneten Umständen austauschbar sind und dass die hier beschriebenen Ausführungsformen der Erfindung in anderer Reihenfolge als hier beschrieben verwendet werden können. Falls sich die Begriffe auf Schritte einer Methode, eines Verfahrens oder einer Verwendung beziehen, gibt es keine Zeit- oder Zeitintervallkohärenz zwischen den Schritten, d.h. die Schritte können gleichzeitig ausgeführt werden oder es können Zeitintervalle von Sekunden, Minuten, Stunden, Tagen, Wochen usw. zwischen solchen Schritten vorliegen, sofern nichts anders angegeben ist.

Es versteht sich, dass diese Erfindung nicht auf die hier beschriebenen speziellen Verfahren, Protokolle usw. beschränkt ist, da diese variieren können. Es versteht sich auch, dass die hierin verwendete Terminologie nur dem Zweck dient, bestimmte Ausführungsformen zu beschreiben, und nicht dazu gedacht ist, den Umfang der vorliegenden Erfindung einzuschränken, der nur durch die beigefügten Ansprüche begrenzt wird.

Die Zeichnungen sind als schematische Darstellungen zu betrachten und in den Zeichnungen dargestellte Elemente sind nicht notwendigerweise maßstabsgetreu dargestellt. Vielmehr sind die verschiedenen Elemente so dargestellt, dass ihre Funktion und ihr allgemeiner Zweck für einen Fachmann offensichtlich werden. Sofern nicht anders definiert, haben alle hierin verwendeten technischen und wissenschaftlichen Begriffe die gleichen Bedeutungen, wie sie üblicherweise von einem Durchschnittsfachmann verstanden werden.

Wie oben ausgeführt, eignet sich das erfindungsgemäße Verfahren zur homogenen Bestimmung der ADAMTS-13 Aktivität in einer biologischen Probe. Der Begriff "homogene Bestimmung", wie hier verwendet, bezieht sich auf die Durchführung der Analyse in homogener Phase. Dieser Ansatz ist schnell und unkompliziert, da keine Trennung von gebundenen und nicht gebundenen Substanzen bzw. keine Waschschritte notwendig sind. Bei der homogenen Bestimmung erfolgt ein "Inkontaktbringen" der Probe mit weiteren Reaktanten vorzugsweise in einer flüssigen Phase, beispielsweise in einem geeigneten Puffersystem.

Typischerweise ist die ADAMTS-13 Aktivität abhängig von Zink- und Calcium-Ionen. Deren Anwesenheit im Reaktionsgemisch ist bei der Durchführung des vorliegenden Verfahrens bevorzugt. Weitere optimale Bedingungen sind z.B. ein pH-Wert von 6 (±1), eine niedrige Ionenstärke, 1-10 mM Calcium-Ionen und/oder 0,01-1 mM Zink-Ionen im Reaktionsgemisch. In weiteren Ausführungsformen ist vorgesehen, dass Barium-Ionen anwesend sind, insbesondere bei Nutzung von Citrat-Plasma, da dadurch die ADAMTS-13-Aktivität zusätzlich über die Freisetzung von Citrat-gebundenem Calcium stimuliert wird. In besonderen Ausführungsformen sollten pH 7.4 und 150 mM NaCl vermieden werden, da sich unter diesen Bedingungen die Aktivität auf ~75% erniedrigt. Das Verfahren sieht vorzugsweise eine Inkubation bei +37°C vor.

Die "ADAMTS-13 Aktivität", wie hierin beschrieben, bezieht sich auf die "Desintegrin- und Metalloproteinase mit Thrombospondin-Typ-1-Motiv, Mitglied 13" und wird auch als "von Willebrand-Faktor-spaltende Protease" (VWFCP) bezeichnet. ADAMTS-13 ist eine Protease, die in Gegenwart von Zink, Calcium, Barium und anderen Metallionen aktiviert wird. Es handelt sich hierbei um ein Plasmaprotein, das den von Willebrand Faktor (VWF) an der Peptidbindung zwischen den Aminosäureresten Y1605 und M1606 spaltet und so seine Größe und damit auch die Adhäsion für Thrombozyten limitiert. Bei verminderter ADAMTS-13-Aktivität, entweder durch vererbte Mutationen im ADAMTS-13-Gen oder beispielsweise durch die Entwicklung von Autoantikörpern, kann die Anhäufung von sehr großen VWF-Multimeren zur Thrombozytenaggregation und damit zu mikrovaskulären Thrombosen führen. Dies entspricht dem potenziell tödlichen Syndrom der thrombotisch-thrombozytopenischen Purpura (TTP). Im Rahmen der vorliegenden Erfindung wird die Aktivität von ADAMTS-13 im Kontext der Spaltung von VWF gemessen.

Der "von Willebrand Faktor" oder "VWF" ist ein großes multimeres Plasmaglykoprotein, das für die Aufrechterhaltung der Hämostase von entscheidender Bedeutung ist, da es sowohl als Träger des antihämophilen Faktors als auch als Thrombozyten-Gefäßwand-Vermittler im Blutgerinnungssystem fungiert, indem es vor allem die Bindung und Adhäsion zirkulierender Thrombozyten an Stellen von Gefäßverletzungen vermittelt. Mutationen im VWF-Gen oder Defizite in diesem Protein führen zur von-Willebrand-Krankheit (VWD). VWF wird typischerweise von Endothelzellen und Megakaryozyten exprimiert. Es werden 250 kDa-Monomere synthetisiert, die einer intrazellulären Prozessierung, Glykosylierung, Multimerisierung und Entfernung von Propeptiden unterzogen werden, was schließlich zur Bildung reifer VWF-Multimere führt. Die Größe des VWF-Multimers wird durch die ADAMTS-13 Protease reguliert, die, wie oben ausgeführt, das VWF-Protein an einer bestimmten, singulären Stelle in der A2-Domäne des VWF-Proteins spaltet.

Das VWF-Monomer ist ein Protein mit 2050 Aminosäuren. Das Monomer enthält eine Reihe spezifischer Domänen mit bestimmten Funktionen. Das Monomer enthält u.a. eine D'/D3-Domäne, die an den Gerinnungs-Faktor VIII bindet; eine A1-Domäne, die an den GPIb-Rezeptor der Blutplättchen, Heparin und möglicherweise Kollagen bindet; eine A2-Domäne, die sich partiell entfalten muss, um die nicht exponierte Spaltstelle für ADAMTS-13 freizulegen; eine A3-Domäne, die an Kollagen bindet; eine C4-Domäne, in der ein RGD-Motiv an das Thrombozytenintegrin αIIbβ3 bindet, wenn dieses aktiviert ist; eine "Cystin-Knoten"-Domäne am C-terminalen Ende des Proteins. Die Monomere werden nach der Expression typischerweise N-glykosyliert und im endoplasmatischen Retikulum zu Dimeren und im Golgi-Apparat durch Vernetzung von Cysteinresten über Disulfidbindungen zu Multimeren angeordnet.

Die von der vorliegenden Erfindung erfassten VWF-Polypeptidsequenzen beziehen sich auf die kanonische humane Version der VWF Isoform 1, welche mit der Hinterlegungsnummer UniProtKB-P04275-1 (VWF_HUMAN) unter https://www.uniprot.org abgerufen werden kann. SEQ ID NO: 25 entspricht der kanonischen humanen Version der VWF Isoform 1. Die A2-Domäne, in welcher sich die ADAMTS-13 Spaltstelle befindet, reicht von Position 1498 bis Position 1668 der genannten kanonischen VWF Isoform 1 (P04275-1). SEQ ID NO: 24 entspricht der A2-Domäne von VWF und umfasst die Positionen D1498 bis R1668.

Der hierin verwendete Begriff "von Willebrand Faktor (VWF)-Polypeptid mit einer ADAMTS-13 Spaltstelle" bezieht sich vornehmlich auf die A2-Domäne, welche die ADAMTS-13-Spaltstelle zwischen Y1605 und M1606 umfasst oder ein Fragment davon, wobei das Fragment die ADAMTS-13-Spaltstelle zwischen Y1605 und M1606 (Tyr1605 und Met1606; bezogen auf die o.g. kanonische Sequenz) umfasst. Das Fragment besitzt eine Konformation, die es erlaubt, von ADAMTS-13 an der Spaltstelle zwischen Y1605 und M1606 proteolytisch gespalten zu werden, d.h. es weist eine funktionelle ADAMTS-13 Spaltstelle auf.

Das VWF-Polypeptid kann unterschiedliche Längen aufweisen und im Vergleich zur o.g. kanonischen Sequenz eine oder mehrere Aminosäuremodifikationen besitzen.

So kann das VWF-Polypeptid beispielsweise eine Länge von 45 bis 171 Aminosäuren aufweisen, beispielsweise von 65 bis 171 Aminosäuren, von 45 bis 110 Aminosäuren, von 45 bis 76 Aminosäuren, von 45 bis 82 Aminosäuren, von 50 bis 73 Aminosäuren oder von 50 bis 70 Aminosäuren. Weiter bevorzugt sind Längen von 70 bis 82 Aminosäuren, von 72 bis 110 Aminosäuren oder von 70 bis 76 Aminosäuren. In einer weiteren Ausführungsform hat das VWF-Polypeptid eine Länge von 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114 oder 115 Aminosäuren.

Das VWF-Polypeptid mit einer ADAMTS-13 Spaltstelle kann eine oder mehrere Aminosäuremodifikationen aufweisen. Die Modifikationen können Aminosäureaustausche, -deletionen oder -additionen sein, oder chemische Veränderungen der Aminosäuren. Diese Modifikationen können, in bestimmten Ausführungsformen, einen Einfluss auf die Spaltbarkeit und Spaltungsgeschwindigkeit durch ADAMTS-13 haben; sie können das Polypeptid in seiner Konformation verändern, seine Faltungsmöglichkeiten beeinflussen oder eine chemische Veränderung umfassen, die bei einer Detektion hilfreich sind.

Die Modifikationen betreffen in bevorzugten Ausführungsformen die Positionen 1599, 1610 und 1617 von SEQ ID NO: 25. Weiter bevorzugt ist, dass die Aminosäure an Position 1599 von SEQ ID NO: 25 von Q zu K verändert ist und/oder dass die Aminosäure an Position 1610 von SEQ ID NO: 25 von N zu C verändert ist und/oder dass die Aminosäure an Position 1617 von SEQ ID NO: 25 von K zu R verändert ist.

In alternativen Ausführungsformen können eine oder mehrere Aminosäuren zu 2,3-Diaminopropionsäure (A2pr) verändert sein. Es ist besonders bevorzugt, dass die Aminosäure an Position 1599 von SEQ ID NO: 25 von Q zu 2,3-Diaminopropionsäure (A2pr) verändert ist und/oder die Aminosäure an Position 1610 von SEQ ID NO: 25 von N zu 2,3-Diaminopropionsäure (A2pr) verändert ist. In alternativen Ausführungsformen können die zu A2pr veränderten Aminosäuren mit einer 2-(N-Methylamino)benzoylgruppe und/oder einer 2,4-Dinitrophenylgruppe modifiziert werden. Es ist weiter bevorzugt, dass die an Position 1599 veränderte Aminosäure mit einer 2-(N-Methylamino)benzoylgruppe (Nma) modifiziert wird und dass die an Position 1610 veränderte Aminosäure mit einer 2,4-Dinitrophenylgruppe (Dnp) modifiziert wird. In dieser Kombination stellen die modifizierten Aminosäuren ein FRET-System dar bei dem nach Anregung der 2-(N-Methylamino)benzoylgruppe mit Strahlung der Wellenlänge 340 nm Fluoreszenzresonanzenergie auf den sog. Quencher, d.h. die 2,4-Dinitrophenylgruppe übertragen wird.

Bevorzugterweise umfasst ein VWF-Polypeptid eine der Sequenzen der SEQ ID NO: 1 bis 24 des Sequenzprotokolls bzw. wie unten in Tabelle 1 wiedergegeben.

**Tabelle 1**

| SEQ ID NO: | Sequenz (N-Term bis C-Term) | Anmerkung |
|---|---|---|
| 1 | DREQAPNLVYMVTGNPASDEIKRLPGDIQVVPIGVGPNAN VQELERIGWPNAPILIQDFETLPREAPDLVLQR | VWF73 (D1596-R1668) |
| 2 | DRE - A2pr (Nma) - APNLVYMVTG - A2pr (Dnp) - PASDEIKRLPGDIQVVPIGVGPNANVQELER IGWPNAPILIQDFETLPREAPDLVLQR | VWF73 (FRET) |
| 3 | DREKAPNLVYMVTGNPASDEIKRLPGDIQVVPIGVGPNAN VQELERIGWPNAPILIQDFETLPREAPDLVLQR | Mutiertes VWF73 (Q1599) |
| 4 | SQGDREQAPNLVYMVTGNPASDEIKRLPGDIQVVPIGVGP NANVQELERIGWPNAPILIQDFETLPREAPDLVLQR | VWF7 6 |
| 5 | SQGDREKAPNLVYMVTGNPASDEIKRLPGDIQVVPIGVGP NANVQELERIGWPNAPILIQDFETLPREAPDLVLQR | Mutiertes VWF76 (Q1599) |
| 6 | SQGDRE - A2pr (Nma) - APNLVYMVTG - A2pr (Dnp) - PASDEIKRLPGDIQVVPIGVGPNANVQELER IGWPNAPILIQDFETLPREAPDLVLQR | VWF76 (FRET) |
| 7 | QGDREQAPNLVYMVTGNPASDEIKRLPGDIQVVPIGVGPN ANVQELERIGWPNAPILIQDFETLPREAPDLVLQR | VWF75 |
| 8 | QGDREKAPNLVYMVTGNPASDEIKRLPGDIQVVPIGVGPN ANVQELERIGWPNAPILIQDFETLPREAPDLVLQR | Mutiertes VWF75 (Q1599) |
| 9 | QGDRE - A2pr (Nma) - APNLVYMVTG - A2pr (Dnp) - PASDEIKRLPGDIQVVPIGVGPNANVQELER IGWPNAPILIQDFETLPREAPDLVLQR | VWF75 (FRET) |
| 10 | GDREQAPNLVYMVTGNPASDEIKRLPGDIQVVPIGVGPNA NVQELERIGWPNAPILIQDFETLPREAPDLVLQR | VWF74 |
| 11 | GDREKAPNLVYMVTGNPASDEIKRLPGDIQVVPIGVGPNA NVQELERIGWPNAPILIQDFETLPREAPDLVLQR | Mutiertes VWF74 (Q1599) |
| 12 | GDRE - A2pr (Nma) - APNLVYMVTG - A2pr (Dnp) - PASDEIKRLPGDIQVVPIGVGPNANVQELER IGWPNAPILIQDFETLPREAPDLVLQR | VWF74 (FRET) |
| 13 | REQAPNLVYMVTGNPASDEIKRLPGDIQVVPIGVGPNANV QELERIGWPNAPILIQDFETLPREAPDLVLQR | VWF72 |
| 14 | REKAPNLVYMVTGNPASDEIKRLPGDIQVVPIGVGPNANV QELERIGWPNAPILIQDFETLPREAPDLVLQR | Mutiertes VWF72 (Q1599) |
| 15 | RE - A2pr (Nma) - APNLVYMVTG - A2pr (Dnp) - PASDEIKRLPGDIQVVPIGVGPNANVQELER IGWPNAPILIQDFETLPREAPDLVLQR | VWF72 (FRET) |
| 16 | EQAPNLVYMVTGNPASDEIKRLPGDIQVVPIGVGPNANVQ ELERIGWPNAPILIQDFETLPREAPDLVLQR | VWF71 |
| 17 | EKAPNLVYMVTGNPASDEIKRLPGDIQVVPIGVGPNANVQ ELERIGWPNAPILIQDFETLPREAPDLVLQR | Mutiertes VWF71 (Q1599) |
| 18 | E - A2pr (Nma) - APNLVYMVTG - A2pr (Dnp) - PASDEIKRLPGDIQVVPIGVGPNANVQELER IGWPNAPILIQDFETLPREAPDLVLQR | VWF71(FRET) |
| 19 | QAPNLVYMVTGNPASDEIKRLPGDIQVVPIGVGPNANVQE LERIGWPNAPILIQDFETLPREAPDLVLQR | VWF70 |
| 20 | GAPNLVYMVTGCPASDEIRRLPGDIQVVPIGVGPNANVQE LERIGWPNAPILIQDFETLPREAPDLVLQR | Mutiertes VWF70 (Q1599, N1610 und K1617) |
| 21 | A2pr (DyLIght 633) - APNLVYMVTG - A2pr (IRDye QC-1) - PASDEIRRLPGDIQVVPIGVGPNAN VQELERIGWPNAPILIQDFETLPREAPDLVLQR | VWF70(Fluor ophor) |
| 22 | DHSFLVSQGDREQAPNLVYMVTGNPASDEIKRLPGDIQVV PIGVGPNANVQELERIGWPNAPILIQDFETLPREAPDLVL QR | VWF82 (D1587-R1668) |
| 23 | EAQSKGDILQRVREIRYQGGNRTNTGLALRYLSDHSFLVS QGDREQAPNLVYMVTGNPASDEIKRLPGDIQVVPIGVGPN ANVQELERIGWPNAPILIQDFETLPREAPDLVLQR | VWF115 (E1554-R1668) |
| 24 | DVAFVLEGSDKIGEADFNRSKEFMEEVIQRMDVGQDSIHV TVLQYSYMVTVEYPFSEAQSKGDILQRVREIRYQGGNRTN TGLALRYLSDHSFLVSQGDREQAPNLVYMVTGNPASDEIK RLPGDIQVVPIGVGPNANVQELERIGWPNAPILIQDFETL PREAPDLVLQR | VWF-A2 (D1498-R1668) |

Der Begriff "inhibierter Reporterenzymkomplex", wie hierin verwendet, bezieht sich auf einen Komplex aus einem Reporterenzym und einem VWF-Polypeptid mit einer ADAMTS-13 Spaltstelle, der zur Signalgenerierung im erfindungsgemäßen Verfahren dient. Der inhibierte Reporterenzymkomplex ist durch die enzymatische Spaltung des VWF-Polypeptids durch ADAMTS-13 aktivierbar und ermöglicht so den Nachweis einer ADAMTS-13 Aktivität in einer Probe. Das infolge der ADAMTS-13-vermittelten Spaltung des VWF-Polypeptids aktivierte Reporterenzym setzt in Abhängigkeit von der Anwesenheit bzw. Menge der ADAMTS-13 Aktivität ein Reporterenzymsubstrat um, was nachweisbar ist. Die gemessene Menge des umgesetzten Reporterenzymsubstrats ist proportional zur ADAMTS-13 Aktivität in der Probe. Der Nachweis kann auf unterschiedlichen Wegen und mit unterschiedlichen Verfahren erfolgen. Bevorzugt sind hierbei eine spektrophotometrische Messung, eine luminometrische Messung, eine Fluoreszenzbestimmung und eine elektrochemische Detektion. Die Art und Umsetzung des Nachweises hängt vom Reporterenzym und dem verwendeten Reporterenzymsubstrat ab.

Nachweisverfahren können mit Hilfe einer Kontrollprobe und/oder einer Kalibrierungsprobe geeicht bzw. kalibriert werden. Reporterenzyme können in ihrer nativen Form, d.h. mit einer Wildtyp-Aminosäuresequenz verwendet werden, oder sie können gentechnisch verändert worden sein. So kann beispielsweise die Aminosäuresequenz an bestimmte Gegebenheiten angepasst werden, z.B. kann die Reporterenzymsubstratbindung optimiert werden, oder die Exprimierbarkeit des Proteins in bestimmten Wirtsorganismen kann beeinflusst werden. Weiterhin kann über die Anwesenheit bzw. Einführung von zusätzlichen Aminosäureresten wie beispielsweise Cystein, Lysin, Asparaginsäure oder Glutaminsäure eine kovalente Verknüpfbarkeit mit Interaktoren erreicht werden.

In bevorzugten Ausführungsformen ist das Reporterenzym eine Malat-Dehydrogenase, eine Beta-Galaktosidase, eine alpha-Amylase oder eine Glukose-6-Phosphat-Dehydrogenase.

Die Malat-Dehydrogenase (MDH) ist ein Enzym, das Malat unter Verbrauch von NAD⁺ zu Oxalessigsäure dehydriert. Die Aktivität von MDH wird typischerweise dadurch bestimmt, dass die Produktion von NADH in der MDH-katalysierten Reaktion Malat + NAD⁺ → Oxalessigsäure + NADH gemessen wird. Eine quantifizierende Messung der Reaktion kann beispielsweise spektrophotometrisch durch direkte Messung bei 340 nm erfolgen, oder nach einer nachgelagerten Farbreaktion des NADH mit einem Farbstoff colorimetrisch, d.h. über die Reduktion eines Reporterfarbstoffs und die Bestimmung der Absorption bei 450 nm durchgeführt werden. Alternativ können neben NAD+ auch NADP+, Thio-NAD+ oder Thio-NADP+ verwendet werden.

beta-Galaktosidase ist ein Enzym, das eine beta-glycosidische Bindung zwischen der Galaktose und ihrem organischen Bindungspartner hydrolysiert. Für die Messung der beta-Galactosidase wird typischerweise das farblose Reporter-Substrat o-Nitrophenyl-β-D-galactopyranosid (ONPG) verwendet, das in Galaktose und das Chromophor o-Nitrophenol umgewandelt wird, das spektrophotometrisch bei 420 nm quantifiziert werden kann.

Glucose-6-phosphat-Dehydrogenase (G6PDH) ist ein Enzym, das Glucose-6-phosphat unter Verwendung von NAD+ in 6-Phosphogluconolacton und NADH umwandelt. Eine quantifizierende Messung der Reaktion kann beispielsweise spektrophotometrisch durch direkte Messung von NADH bei 340 nm erfolgen oder nach einer nachgelagerten Farbreaktion des NADH mit einem Farbstoff colorimetrisch, d.h. über die Reduktion eines Reporterfarbstoffs und die Bestimmung der Absorption bei 450 nm, durchgeführt werden. Alternativ können neben NAD+ auch NADP+, thio-NAD+ oder thio-NADP+ verwendet werden. Weitere Details kann der Fachmann geeigneten Literaturquellen entnehmen.

Besonders bevorzugt ist die Verwendung von G6PDH als Reporterenzym.

Der Begriff "Reporterenzymsubstrat", wie hierin verwendet, bezieht sich auf ein Molekül, das eine Affinität zum aktiven Zentrum des Reporterenzyms besitzt und von diesem in einer katalysierten Reaktion umgesetzt werden kann. Das Reporterenzymsubstrat ist vom gewählten Reporterenzym abhängig. Geeignete Reporterenzymsubstrate für die Malat-Dehydrogenase (MDH) bzw. die Glucose-6-phosphat-Dehydrogenase (G6PDH) sind NAD+, NADP+, thio-NAD+ oder thio-NADP+, die in NADH, NADPH, thio-NADH oder thio-NADPH umgewandelt werden. Ein geeignetes Reporterenzymsubstrat für die beta-Galaktosidase ist ONPG, das u.a. in o-Nitrophenol umgewandelt wird. Weitere Reporterenzymsubstrate im Kontext von bestimmten Reporterenzymen sind dem Fachmann bekannt.

Zusätzlich zum Reporterenzymsubstrat können noch einer oder mehrere Cofaktoren in dem Reaktionsgemisch verwendet werden. Der Begriff "Cofaktor" bezieht sich im Rahmen der vorliegenden Erfindung auf eine niedermolekulare Substanz, die zum Ablauf einer biochemischen Reaktion beiträgt. Cofaktoren können beispielsweise kovalent oder nicht-kovalent an das Reporterenzym gebunden oder in dieses eingelagert sein. Beispiele für Cofaktoren sind ATP, ADP oder Metallionen, beispielsweise von Zink, Calcium, Barium, Magnesium oder Kupfer.

Die Bereitstellung eines geeigneten inhibierten Reporterenzymkomplex, der ein von Willebrand Faktor (VWF)-Polypeptid mit einer ADAMTS-13 Spaltstelle und ein Reporterenzym umfasst und der durch die enzymatische Spaltung des VWF-Polypeptids durch ADAMTS-13 aktivierbar ist, kann durch Kopplung des VWF-Polypeptides an das Reporterenzym über eine kovalente Bindung erfolgen. Im Reporterenzym kann eine kovalente Bindung beispielsweise über die Anwesenheit einer Thiol- oder Sulfhydrylgruppe in einem Cysteinrest, die Anwesenheit einer Aminogruppe in einem Lysinrest oder die Anwesenheit einer Carboxygruppe in einem Asparaginsäurerest oder Glutaminsäurerest herbeigeführt werden. Typischerweise erfolgt die Kopplung über aktivierende Moleküle. So können beispielsweise Cysteine mit Maleimiden, Disulfiden, Iodacetamid, Haloacetylen, Aziridinen, Acryloylen, Arylierungsmitteln, Vinylsulfonen, Pyridyl- und anderen Disulfiden selektiv reagieren. Aminosäuren mit primären Aminen an der Seitenkette wie Lysin können durch Succinimidester (N-Hydroxysuccinimid, Sulfosuccinimid- oder andere Succinimidylester) oder bestimmte Isothiocyanate, wie PITC oder FITC, aktiviert und verknüpft werden. Carboxygruppen können durch eine Aktivierung mit Carbodiimiden an Amingruppen gekoppelt werden. Weitere Möglichkeiten sind eine reduktive Alkylierung und eine Tosylierung. Weiterhin können photoreaktive Moleküle, wie z.B. Arylazide oder Diazirine, als reaktive Gruppe einer kovalenten Kopplung an Proteine verwendet werden. Dabei wird die Reaktion mit UV-Bestrahlung ausgelöst. Die kovalente Bindung wird vorzugsweise mit einer Aminogruppe am N-Terminus des VWF-Polypeptids oder mit einer Carboxygruppe am C-Terminus des VWF-Polypeptids durchgeführt.

In weiteren bevorzugten Ausführungsformen wird die Verknüpfung über einen Spacer vorgenommen. Der Begriff "Spacer" bezieht sich auf jede Struktur einer Molekülkette, die das Reporterenzym kovalent mit dem VWF-Polypeptid verknüpft. Der Spacer kann aus Kohlenstoffatomen und Stickstoffatomen und/oder Sauerstoffatomen und/oder Wasserstoffatomen bestehen. Beispielsweise kann die Molekülkette eine Gesamtzahl an Kohlenstoffatomen, Stickstoffatomen und Sauerstoffatomen, die in der Hauptkette enthalten sind, von mindestens 3, vorzugsweise zwischen 3 Atomen und 100 Atomen, stärker bevorzugt zwischen 3 Atomen und 30 Atomen aufweisen, und noch mehr bevorzugt zwischen 3 Atomen bis 20 Atomen, z.B. 3, 4, 5, 6, 7, 8, 9, 10, 11, 12, 12, 14, 15, 16, 17, 18, 19, 20 Atome. Besonders bevorzugt ist eine Länge von 3 bis 10 Atomen. In einer anderen Ausführungsform kann die Länge der Hauptkette des Spacers etwa 0,4 nm bis etwa 100 nm und bevorzugter etwa zwischen 0,6 nm und 30 nm betragen, z.B. 0,6, 0,8, 1, 2, 4, 8, 10, 20, 25 oder 30 nm. In weiteren bevorzugten Ausführungsformen ist der Spacer ein Peptidspacer, z.B. ein Glycin-Spacer mit zwischen 2 und 10 Gylcinresten. In weiteren Ausführungsformen kann der Spacer ein hydrophiler Spacer auf Ethylenglycol-Basis sein.

Das VWF-Polypeptid kann am freien, d.h. an dem nicht an das Reporterenzym gekoppelten Terminus mit einem molekularen Epitop-Tag versehen sein. Das molekulare Epitop-Tag kann am N-Terminus oder am C-Terminus angebracht sein. Eine Anbringung am C-Terminus des VWF-Polypeptids ist bevorzugt. Der Begriff "molekulares Epitop-Tag", wie hierin verwendet, bezieht sich auf ein kleines organchemisches Molekül oder eine kurze Aminosäuresequenz, die als Epitop für eine Antikörperbindung oder als Interaktor für molekulare Interaktionen mit einem anderen Bindungspartner verwendet werden können. Beispiele für molekulare Epitop-Tags, die im Rahmen der Erfindung Verwendung finden können, sind Peptid-Tags wie das ALFA-Tag, Avi-Tag, C-Tag, Calmodulin-Tag, Polyglutamat-Tag, Polyarginine-Tag, E-Tag, FLAG-Tag, HA-Tag, His-Tag, Gly-His-Tag, Myc-Tag, NE-Tag, Rho1D4-Tag, S-Tag, SBP-Tag, Softag 1-Tag, Softag 3-Tag, Spot-Tag, Strep-Tag, T7-Tag, TC-Tag, Ty-Tag, V5 Tag, VSV-Tag, Xpress Tag. Weitere Beispiele umfassen Protein-Tags wie das BCCP-Tag, Glutathione-S-transferase (GST)-Tag, das Green fluorescent protein (GFP)-Tag, Halo-Tag, SNAP-Tag, CLIP-Tag, HUH-Tag, Maltose binding protein-Tag, Thioredoxin-Tag, Fc-Tag, CRDSAT-Tag oder HiBiT-Tag. Besonders bevorzugt sind die Tags Biotin, FITC, Streptavidin, GST, His, Flag, ACP, ELK16, HA oder MBP. Alternativ kann das Epitop-Tag in Form eines DNA-Oligonukleotides vorliegen, das mit einem komplementären DNA-Strang hybridisiert.

Wie in Fig. 1 bzw. Fig. 2 illustriert, basiert das erfindungsgemäße Verfahren darauf, dass eine Inhibierung des Reporterenzyms, die durch die Kopplung des nicht gespaltenen VWF-Polypeptids verursacht wird, bevorzugt eines VWF-Polypeptids entsprechend einer der SEQ ID NO: 1 bis SEQ ID NO: 24, und die dazu beiträgt, dass das Reporterenzym nicht bzw. nicht quantitativ mit dem Reporterenzymsubstrat interagieren kann, durch die proteolytische ADAMTS-13 Aktivität, d.h. durch die durch ADAMTS-13 verursachte Spaltung des gekoppelten VWF-Polypeptids an der Spaltstelle zwischen Y1605 und M1606, aufgehoben wird. Durch die Trunkierung des gekoppelten VWF-Polypeptids auf ein Fragment, das vorzugsweise mit Y1605 endet, alternativ auch mit M1606 enden kann, wird die Blockierung des aktiven Zentrums des Reporterenzyms aufgehoben. Das Reporterenzym kann bei Vorliegen des Reporterenzymsubstrates im Reaktionsgemisch dieses quantitativ umsetzen. Die Umsetzung des Substrates kann durch Messung der Reaktionsprodukte gemessen werden. Diese Messung ist eine quantitative Bestimmung der vorhandenen Menge an ADAMTS-13 in der eingesetzten Probe. Der Begriff "quantitative Bestimmung", wie hierin benutzt, bezieht sich auf die direkte Proportionalität zwischen der Menge an ADAMTS-13 und der Menge von dem infolge der proteolytischen Spaltung des gekoppelten VWF-Polypeptides aktivierten Reporterenzyms umgesetzten Reporterenzymsubstrates. Dadurch ist es möglich, die Menge an vorhandener ADAMTS-13 Aktivität in dem Reaktionsgemisch abzubilden.

In einer weiteren Ausführungsform, die beispielsweise in Fig. 3 illustriert ist, wird die durch das gekoppelte VWF-Polypeptid verursachte Inhibierung durch die Bindung eines Bindungspartners an das distale Ende des VWF-Polypeptids verstärkt. Das "distale" Ende des gekoppelten VWF-Polypeptids ist als das dem Kopplungsort mit dem Reporterenzym entgegengesetzte Ende des Polypeptides bzw. als weiter entfernter Bereich des Polypeptides, vom Kopplungsort aus betrachtet, zu verstehen. Dieses Ende ist vorzugweise der nicht gekoppelte C-Terminus des Polypeptides, kann alternativ aber auch der nicht gekoppelte N-Terminus sein. Der Begriff "Bindungspartner", wie hierin verwendet, bezieht sich auf alle dem Fachmann bekannten Einheiten, die den genannten distalen Bereich des Polypeptides spezifisch binden können. In weiteren spezifischen Ausführungsformen kann der Bindungsort der Bindungspartner an anderen Stellen bzw. in anderen Regionen des gekoppelten VWF-Polypeptides liegen, z.B. ein Epitop im nicht distalen Bereich sein. Bevorzugt ist, dass ein solcher Bindungsort distal der ADAMTS-13 Spaltstelle zwischen Y1605 und M1606 im VWF-Polypeptid liegt. Es ist weiter bevorzugt, dass am distalen Ende ein molekulares Epitop-Tag vorhanden ist, beispielsweise wie oben definiert, das vorzugsweise von einem Antikörper oder einem anderen Bindungspartner erkannt werden kann. In weiteren bevorzugten Ausführungsformen ist ein terminales Epitop vorhanden, das von einem Antikörper erkannt werden kann.

Die zusätzliche Bindung eines Bindungspartners an das VWF-Polypeptid, vorzugsweise an einen Bereich des VWF-Polypeptids, der in Gegenwart von ADAMTS-13 von dem Reporterenzymkomplex abgespalten wird, verstärkt die Inhibierung des Reporterenzyms bei fehlender proteolytischer Spaltung des VWF-Polypeptids.

Ein Bindungspartner für die Verstärkung der Inhibierung des Reporterenzymkomplexes umfasst, zum Beispiel, spezifisch bindende Antikörper, Aptamere oder Affimere. Weiterhin sind künstliche Antikörper-Mimetika umfasst, also polymere organische oder anorganische Strukturen, die ein Zielmolekül spezifisch binden, typischerweise mit einer Bindungsaffinität im Bereich eines Antikörpers.

Der Begriff "Antikörper", wie er hierin verwendet wird, bezieht sich auf Immunglobulinmoleküle und immunologisch aktive Teile von Immunglobulinmolekülen, d.h. Moleküle, die eine Antigenbindungsstelle enthalten, die ein Antigen immunspezifisch bindet. Die Immunglobulinmoleküle der Erfindung können von jedem Typ (z. B. IgG, IgE, IgM, IgD, IgA und IgY), jeder Klasse (z. B. IgG1, IgG2, IgG3, IgG4, IgA1 und IgA2) oder Unterklasse von Immunglobulinmolekülen sein. In weiteren Ausführungsformen umfassen Antikörper der Erfindung polyklonale, monoklonale, multispezifische, murine, menschliche, humanisierte oder chimäre Antikörper, oder ganze Immunglobulinmoleküle.

Der Begriff "Aptamer", wie hierin verwendet, bezieht sich auf ein Peptid oder eine Nukleinsäure, das/die spezifisch an ein Zielepitop bindet. Aptamere werden typischerweise aufgrund ihrer selektiven Bindungseigenschaften aus Pools von Nukleinsäuren und/oder Peptiden ausgewählt. Ein "Nukleinsäureaptamer" ist ein kurzes Nukleinsäuremolekül, z.B. RNA, DNA, PNA, CNA, HNA, LNA oder ANA oder ein beliebiges anderes geeignetes, dem Fachmann bekanntes Nukleinsäureformat, das in der Lage ist, spezifisch an ein Protein-Zielmolekül zu binden. Ein "Peptidaptamer" ist ein kurzes Peptid, das in der Lage ist, mit einem Proteinziel zu interagieren und sich spezifisch daran zu binden. Typischerweise umfassen Peptidaptamere eine variable Peptidschleife, die an beiden Enden an ein Proteingerüst gebunden ist. Es wird angenommen, dass diese doppelte strukturelle Einschränkung die Bindungsaffinität des Peptidaptamers stark erhöht, sodass diese mit der eines Antikörpers vergleichbar ist. Die variable Schleifenlänge kann vorzugsweise aus zehn bis zwanzig Aminosäuren zusammengesetzt sein.

Eine bevorzugte Subform des Peptidaptamers ist das "Affimer". Es handelt sich hierbei um ein künstlich erzeugtes Protein aus der Gruppe der Cystatine. Affimere basieren auf dem Proteaseinhibitor Cystatin A und werden durch Proteindesign an der Moleküloberfläche individuell verändert. Dadurch kann ein spezifisches Affimer an spezifisch gewählte Moleküle binden. Der Cystatin-Anteil dient dabei als Gerüst zur Stabilisierung der Konformation einer beispielsweise in der Oberfläche eingefügten Sequenz, etwa einer alpha-Helix.

Vorteilhafterweise besitzen Affimere eine hohe biologische Halbwertszeit und Thermostabilität und können über einen längeren Zeitraum stabil verwendet werden.

Die Anwesenheit eines Bindungspartners, der am distalen Ende des VWF-Polypeptids spezifisch gebunden ist, erhöht die Inhibierungswirkung des gekoppelten VWF-Polypeptides. In spezifischen Ausführungsformen können dadurch relativ kurze VWF-Fragmente, wie oben beschrieben, mit Aminosäurelängen zwischen 45 bis 171 Aminosäuren verwendet werden, welche eine funktionelle ADAMTS-13 Spaltstelle aufweisen.

Der Begriff "Probe", wie hierin verwendet, umfasst Wirbeltier-Körperflüssigkeiten, vorzugsweise Säugetier-Körperflüssigkeiten, weiter bevorzugt menschliche Körperflüssigkeiten. Umfasst sind alle Arten von Körperflüssigkeiten, in denen ADAMTS-13 Aktivität vorhanden sein kann. Bevorzugt sind insbesondere Vollblut, Blutplasma, Blutserum, Speichel, Liquor oder Tränenflüssigkeit.

Ein weiterer Aspekt der Erfindung betrifft ein Kit, umfassend ein erstes Reagenz enthaltend einen inhibierten Reporterenzymkomplex, der ein von Willebrand Faktor (VWF)-Polypeptid mit einer ADAMTS-13 Spaltstelle und ein Reporterenzym umfasst, der durch die enzymatische Spaltung des VWF-Polypeptids durch ADAMTS-13 aktivierbar ist, und ein zweites Reagenz enthaltend ein Reporterenzymsubstrat und optional einen oder mehrere Cofaktoren für das Reporterenzym wie hierin beschrieben. Das Kit kann weiterhin in bestimmten Ausführungsformen Bindungspartner wie Antikörper, Aptamere oder Affimere enthalten. Das Reporterenzym und das VWF-Polypeptid entsprechen den hierin, im Kontext des Verfahrens ausgeführten Definitionen und Beschreibungen bzw. wie in den illustrativen, nicht einschränkenden Figuren erläutert.

Das erfindungsgemäße Kit ist eine diagnostische Zusammensetzung für die in vitro-Bestimmung der ADAMTS-13 Aktivität in einer Probe eines Individuums, z.B. eines Patienten bzw. einer Person, um z.B. einen Mangel an ADAMTS-13 Aktivität bzw. die Höhe der ADAMTS-13 Aktivität bestimmen zu können. Dadurch kann zusätzlich das Vorhandensein einer thrombotisch-thrombozytopenischen Purpura (TTP) oder die Prädisposition für TTP bestimmt werden. Zusätzlich kann das Kit einen Beipackzettel mit Anwendungshinweisen enthalten. Weiterhin kann das Kit eine oder mehrere Positivkontrollen und/oder einen oder mehrere Kalibratoren mit definierter ADAMTS-13 Aktivität enthalten. Ein erfindungsgemäßes Kit kann ferner ein Probenverdünnungsmittel und/oder einen Substratpuffer enthalten.

In einem weiteren Aspekt bezieht sich die vorliegende Erfindung auf die Verwendung eines inhibierten Reporterenzymkomplexes, der ein VWF-Polypeptid mit einer ADAMTS-13 Spaltstelle und ein Reporterenzym umfasst und der durch die enzymatische Spaltung des VWF-Polypeptids durch ADAMTS-13 aktivierbar ist, für die Bestimmung von ADAMTS-13 Aktivität in einer biologischen Probe. Das Reporterenzym und das VWF-Polypeptid entsprechen den hierin, im Kontext des Verfahrens ausgeführten Definitionen und Beschreibungen bzw. wie in den illustrativen, nicht einschränkenden Figuren erläutert. Die Verwendung bezieht sich ebenfalls auf die Benutzung von Bindungspartnern wie Antikörpern, Aptameren oder Affimeren wie oben definiert oder in den nicht einschränkenden Figuren illustriert.

Die folgenden Beispiele und Figuren dienen der Veranschaulichung. Es versteht sich daher, dass die Beispiele und Figuren nicht als einschränkend ausgelegt werden sollen. Der Fachmann kann sich weitere Modifikationen der hier dargelegten Prinzipien klar vorstellen.

### BEISPIEL

### A) VWF73-G6PDH Konjugation zur Herstellung eines inhibierten G6PDH-VWF-Reporterenzymkomplexes

Mit NHS-Ester aktiviertes VWF73-Polypeptid (SEQ ID NO: 1) wurde über NH2-Gruppen der G6PDH an G6PDH (Glucose-6-Phosphat Dehydrogenase aus L.mesenteroides; lyophilisiertes Pulver in 100 mM Natrium Phosphat Puffer (SPB), pH 7.3) kovalent gekoppelt. Hierbei wurden 100 U/mL G6PDH mit 1 mM VWF73-Polypeptid über 4 Stunden bei Raumtemperatur gekoppelt. Überschüssiges VWF73-Polypeptid wurde danach über Gelfiltration mit PD-10 Säulen (Amersham Biosciences) und/oder Zentrifugalfiltration mit z.B. Microcon YM-30 (Millipore) abgetrennt. Alle Schritte erfolgten bei Raumtemperatur.

### B) Erfindungsgemäße Bestimmung der ADAMTS-13-Aktivität

Das erfindungsgemäße Verfahren wurde automatisiert auf einem Atellica COAG 360 System (Siemens Healthineers) abgearbeitet. Dabei wurden 30 µL VWF73-G6PDH-Konjugat (0,5 U/mL) (Reagenz 1) mit 30 µL Probe und mit 50 µL Aktivierungspuffer (Reagenz 2 = 20 mM BIS-TRIS, pH 6,0, 12,5 mM BaCl2, 5 mM ZnCI2, 1,5 mM CaCl2 und 0,05 % Tween-20) vermischt und bei +37 °C für 20 min inkubiert. Danach wurden dem Reaktionsgemisch 130 µL auf +37 °C vortemperierte Substratlösung (Reagenz 3 = 0,018 M Glucose-6-phosphat und 0,018 M NAD+ in 55 mM Tris-HCl Puffer mit 3,3 mM MgCl2, pH 7,8) hinzugegeben, und es wurde die Zunahme der Absorption des Reaktionsgemischs in mOD (infolge der Bildung von NADH + H+ durch Reduktion von NAD+ über die G6PDH-Aktivität) über zwei Minuten bei 340 nm gemessen. Aus den mOD-Messwerten wurde die Reaktionsgeschwindigkeit der enzymatischen Reaktion bestimmt (Delta mOD/min, Δ mOD/min).

Auf diese Weise wurden Citrat-Plasmaproben von fünf gesunden Spendern, von zwei Spendern mit thrombotischthrombozytopenischer Purpura (TTP) und einem Spender mit Autoantikörpern gegen ADAMTS-13, aber ohne klinischen Befund einer TTP, in jeweils drei unabhängigen Reaktionsansätzen untersucht, und es wurde der Mittelwert gebildet (siehe Tabelle 3).

Die Bestimmung der ADAMTS-13 Aktivität in einer Probe erfolgte durch den Vergleich mit einer ADAMTS-13 Standardkurve hergestellt aus einer Verdünnungsreihe von FFP (Fresh Frozen Plasma Pool) aus > 80 Citrat-Plasmen gesunder Spender (Normalplasma) mit 100 % arbiträrer ADAMTS-13 Aktivität verdünnt mit ADAMTS-13 Mangelplasma (0 % - 100 % ADAMTS-13 Aktivität). Jede Verdünnungsstufe wurde in jeweils drei unabhängigen Reaktionsansätzen untersucht, und es wurde der Mittelwert gebildet (siehe Tabelle 2).

**Tabelle 2**

| **Anteil Normalplasma in ADAMTS-13 Mangelplasma; ADAMTS-13 Aktivität (% der Norm)** | **Δ mOD/min Messung 1** | **Δ mOD/min Messung 2** | **Δ mOD/min Messung 3** | **Δ mOD/min Mittelwert** |
|---|---|---|---|---|
| 0 % | 207,9 | 207,8 | 207,3 | **207,7** |
| 5 % | 215,6 | 216,5 | 213,9 | **215,3** |
| 10 % | 221,1 | 221,3 | 218,8 | **220,4** |
| 25 % | 232,2 | 232,7 | 232,6 | **232,5** |
| 50 % | 255,3 | 257,3 | 254,0 | **255,5** |
| 75 % | 285,9 | 287,6 | 285,9 | **286,5** |
| 100 % | 310,2 | 312,1 | 310,9 | **311,1** |

Die Ergebnisse in Tabelle 2 zeigen, dass die Spaltung des VWF73-Polypeptids des VWF73-G6PDH-Konjugats und die damit verbundene Reaktivierung der G6PDH-Aktivität konzentrationsabhängig von der ADAMTS-13-Aktivität erfolgt. Mit zunehmender ADAMTS-13 Aktivität (in % der Norm) steigt die Spaltungsrate des gekoppelten VWF73 Polypeptids, und die damit verbundene Reaktivierung der G6PDH-Aktivität führt zur Zunahme der Absorption des Reaktionsansatzes bei 340 nm Wellenlänge. Die Ergebnisse des Experiments zeigen auch, dass das beschriebene Verfahren eine ADAMTS-13 Aktivität von 5% der Norm noch ausreichend von einer Probe ohne ADAMTS-13 Aktivität (ADAMTS-13 Mangelplasma) unterscheiden kann.

**Tabelle 3**

| **Patientenplasma** | **Δ mOD/min Messung 1** | **Δ mOD/min Messung 2** | **Δ mOD/min Messung 3** | **Δ mOD/min Mittelwert** |
|---|---|---|---|---|
| **Gesunder Spender 01** | 308,9 | 309,5 | 310,9 | **309,8** |
| **Gesunder Spender 02** | 310,1 | 310,9 | 312,3 | **311,1** |
| **Gesunder Spender 03** | 305,1 | 308,7 | 306,1 | **306,6** |
| **Gesunder Spender 04** | 311,9 | 310,9 | 308,8 | **310,5** |
| **Gesunder Spender 05** | 315,1 | 312,3 | 314,1 | **313,8** |
| **TTP Patient 01** | 217,6 | 219,1 | 218,2 | **218,3** |
| **TTP Patient 02** | 220,1 | 215,4 | 213,9 | **216,5** |
| **Patient mit ADAMTS-13-Autoantikörpern** | 227,3 | 228,4 | 228,9 | **228,2** |

Tabelle 3 zeigt, dass das beschriebene Verfahren TTP Patientenproben (TTP Patient 01 mit 9 % der Norm und TTP Patient 02 mit 7 % der Norm) oder Patientenproben mit ADAMTS-13 Autoantikörpern (Patient mit 21 % der Norm) mit erniedrigter ADAMTS-13 Aktivität deutlich von Proben von gesunden Blutspendern differenzieren kann. Eine starke Verminderung der ADAMTS-13-Aktivität im Blut (< 5-10% Restaktivität) wird als spezifischer Hinweis auf eine klassische TTP (thrombotisch-thrombozytopenische Purpura) angesehen (angeboren oder erworben).

## Patentansprüche

1. Verfahren zur Bestimmung der ADAMTS-13 Aktivität in einer Probe, das Verfahren umfassend die folgenden Schritte:
a. Bereitstellen eines Reaktionsgemischs durch Vermischen der Probe mit
i. einem inhibierten Reporterenzymkomplex, der ein von Willebrand Faktor (VWF)-Polypeptid mit einer ADAMTS-13 Spaltstelle und ein Reporterenzym umfasst und der durch die enzymatische Spaltung des VWF-Polypeptids durch ADAMTS-13 aktivierbar ist und
ii. einem Reporterenzymsubstrat;
b. Messen der Menge des von dem aktivierten Reporterenzym umgesetzten Reporterenzymsubstrats in dem Reaktionsgemisch,
wobei die gemessene Menge des umgesetzten Reporterenzymsubstrats proportional zur ADAMTS-13 Aktivität in der Probe ist.

2. Verfahren gemäß Anspruch 1, wobei das Reporterenzym Lysozym, Malat-Dehydrogenase, beta-Galaktosidase, alpha-Amylase oder Glukose-6-Phosphat-Dehydrogenase (G6PDH) oder eine funktionelle Variante davon ist.

3. Verfahren gemäß Anspruch 1 oder 2, wobei die Messung des umgesetzten Reporterenzymsubstrats mittels einer spektrophotometrischen, luminometrischen oder elektrochemischen Methode erfolgt.

4. Verfahren gemäß einem der Ansprüche 1 bis 3, wobei der inhibierte Reporterenzymkomplex ein VWF-Polypeptid mit einer ADAMTS-13 Spaltstelle und ein Reporterenzym umfasst, die kovalent miteinander verknüpft sind, vorzugsweise über einen Spacer, weiter bevorzugt über einen Spacer mit 3 bis 10 Atomen.

5. Verfahren nach einem der Ansprüche 1 bis 4, wobei das VWF-Polypeptid die A2 Domäne des VWF umfasst.

6. Verfahren nach einem der Ansprüche 1 bis 4, wobei das VWF-Polypeptid die Aminosäuresequenz einer der SEQ ID NO: 1 bis SEQ ID NO: 24 aufweist.

7. Verfahren nach einem der Ansprüche 1 bis 6, wobei das VWF-Polypeptid C-terminal mit einem Epitop-Tag, vorzugsweise Biotin, FITC, Streptavidin, GST, His, Flag, ACP, ELK16, HA oder MBP, versehen ist.

8. Verfahren nach einem der Ansprüche 1 bis 7, wobei der inhibierte Reporterenzymkomplex zusätzlich einen an das VWF-Polypeptid spezifisch gebundenen Bindungspartner aus der Gruppe Antikörper, Aptamer und Affimer umfasst.

9. Verfahren nach Anspruch 8, wobei der spezifisch gebundene Bindungspartner an ein Epitop-Tag am VWF-Polypeptid gebunden ist.

10. Verfahren nach einem der Ansprüche 1 bis 9, wobei die Probe eine Körperflüssigkeit, vorzugsweise Vollblut, Blutplasma, Blutserum, Speichel, Liquor oder Tränenflüssigkeit ist.

11. Kit, umfassend ein erstes Reagenz enthaltend einen inhibierten Reporterenzymkomplex, der ein von Willebrand Faktor (VWF)-Polypeptid mit einer ADAMTS-13 Spaltstelle und ein Reporterenzym umfasst und der durch die enzymatische Spaltung des VWF-Polypeptids durch ADAMTS-13 aktivierbar ist, und ein zweites Reagenz enthaltend ein Reporterenzymsubstrat und optional einen oder mehrere Cofaktoren für das Reporterenzym.

12. Verwendung eines inhibierten Reporterenzymkomplexes, der ein VWF-Polypeptid mit einer ADAMTS-13 Spaltstelle und ein Reporterenzym umfasst und der durch die enzymatische Spaltung des VWF-Polypeptids durch ADAMTS-13 aktivierbar ist, für die Bestimmung der ADAMTS-13 Aktivität in einer Probe.
